# EUROPÄISCHE PATENTSCHRIFT

(11) **EP 0 548 794 B1**
(45) Veröffentlichungstag und Bekanntmachung des Hinweises auf die Patenterteilung: **09.06.1999**
(21) Anmeldenummer: 92121461.5
(22) Anmeldetag: 17.12.1992
(51) Int. Cl.: A61K 9/16, C08G 73/14, C08G 69/36

(54) **Polyaspartamidderivate als Adsorptionsmittel für Gallensäuren, mit Gallensäuren beladene Polyaspartamidderivate und Verfahren zu deren Herstellung sowie deren Anwendung als Arzneimittel**
Polyaspartamide derivative as adsorbent for bile acids, polyaspartamide loaded with bile acids and preparation and use as a drug
Dérivés de polyaspartamide adsorbant les acides biliaires, dérivés de polyaspartamide chargés d'acides biliaires et leur procédé de préparation et leur utilisation comme médicament

(30) Priorität: 20.12.1991 DE 4142147
(43) Veröffentlichungstag der Anmeldung: 30.06.1993
(73) Patentinhaber: HOECHST AKTIENGESELLSCHAFT, 65926 Frankfurt am Main (DE)
(72) Erfinder: Krone, Volker, Dr., W-6238 Hofheim/Ts. (DE); Walch, Axel, Dr., W-6000 Frankfurt/M. 90 (DE); Müllner, Stefan, Dr., W-6203 Hochheim am Main (DE); Granzer, Ernold, Dr. Dr., W-6233 Kelkheim/Ts. (DE)

(56) Entgegenhaltungen:
- EP-A- 0 274 127
- EP-A- 0 389 079
- EP-A- 0 439 846
- EP-A- 0 459 632
- FR-A- 2 321 898
- POLYMERS FOR ADVANCED TECHNOLOGIES, Bd.1, Nr.5/6, Oktober 1990, SUSSEX, GB Seiten 275 - 285 MARIA DE L. MACHADO ET AL 'Water-soluble Polyamides as Potential Drug Carriers, IV: Amine-functionalized Copolyaspartamides'

## Beschreibung

Die Erfindung betrifft wasserlösliche und -unlösliche Polyaspartamide, Polyaspartamidderivate, die mit Gallensäuren beladen sind, ein Verfahren zu deren Herstellung sowie deren Anwendung als Arzneimittel.

Gallensäuren haben eine wichtige physiologische Funktion bei der Fettverdauung, z. B. als Cofaktoren der pankreatischen Lipasen. Als Endprodukt des Cholesterinstoffwechsels werden sie in der Leber synthetisiert, in der Gallenblase gespeichert und aus dieser durch Kontraktion in den Dünndarm abgegeben, wo sie ihre physiologische Wirkung entfalten.

Der größte Teil der sezernierten Gallensäuren wird über den enterohepatischen Kreislauf wieder zurückgewonnen, über die Mesenterialvenen des Dünndarms und das Pfortadersystem gelangen sie wieder zur Leber zurück. Bei der Rückresorption im Darm spielen sowohl aktive als auch passive Transportprozesse eine Rolle. Im enterohepatischen Kreislauf treten die Gallensäuren sowohl als freie Säuren, aber auch in Form von Glycin- und Taurinkonjugaten in Erscheinung.

Bisher ist bekannt, Gallensäure an nichtresorbierbare, unlösliche, basische vernetzte Polymere (Ionenaustauscherharze = "Resins") zu binden. Als Therapieobjekt werden alle Erkrankungen, bei denen eine Hemmung der Gallensäurerückresorption im Darm, insbesondere im Dünndarm, wünschenswert erscheint, angesehen. Beispielsweise werden die chologene Diarrhoe nach Ileumresektion, oder auch erhöhte Cholesterin-Blutspiegel auf diese Weise behandelt. Im Falle des erhöhten Cholesterin-Blutspiegels kann durch den Eingriff in den enterohepatischen Kreislauf eine Senkung dieses Spiegels erreicht werden. Durch Senkung des im enterohepatischen Kreislauf befindlichen Gallensäurepools wird die entsprechende Neusynthese von Gallensäuren aus Cholesterin in der Leber erzwungen. Zur Deckung des Cholesterinbedarfs in der Leber wird auf das im Blutkreislauf befindliche LDL-Cholesterin (Low Density Lipoprotein) zurückgegriffen, wobei die hepatischen LDL-Rezeptoren in vermehrter Anzahl zur Wirkung kommen. Die so erfolgte Beschleunigung des LDL-Katabolismus wirkt sich durch die Herabsetzung des atherogenen Cholesterinanteils im Blut aus. Bislang stellten diese genannten polymeren, unlöslichen Ionenaustauscher-Harze die einzige Möglichkeit dar, den enterohepatischen Kreislauf hinsichtlich erhöhter Gallensäureausscheidung und daraus folgender Senkung des Cholesterinspiegels zu beeinflussen.

Es zeigt sich nun, daß die Arzneimittel, die auf vernetzten Ionenaustauscherharzen basieren, verschiedene Nachteile aufweisen.

Für die als Arzneimittel in der Verwendung befindlichen "Resins" ist insbesondere eine sehr hohe Tagesdosis einzuhalten. Sie beträgt z. B. für Colestyramin (enthält quartäre Ammoniumgruppen) 12 - 14 g, Höchstdosis 32 g, für Colestipol (enthält sekundäre bzw. tertiäre Aminogruppen) 15 - 30 g.

Ein weiterer Nachteil ist, daß Geschmack, Geruch und die genannte hohe Dosierung die Patienten-Compliance erschweren.

Weiterhin ist bekannt, daß herkömmliche "Resins" Nebenwirkungen zeigen. Diese Nebenwirkungen gehen auf mangelnde Selektivität (z. B. Avitaminosen) zurück, die auch bei der Dosierung simultan gegebener Medikamente berücksichtigt werden müssen, aber auch auf Gallensäureverarmung, die verschiedene gastrointestinale Störungen (Obstipation, Steatorrhoe) unterschiedlichen Grades hervorrufen.

Für beide Präparate wurde eine therapeutische Bedeutung durch Kombination mit anderen hypolipidämisch wirkenden Pharmaka wie Fibraten, HMG-CoA-Reduktase-Inhibitoren, Probucol (vgl. z. B. M. N. Cayen, Pharmac. Ther. 29, 187 (1985) und 8th International Symposium on Atherosclerosis, Rome, Oct. 9 - 13, 1988, Abstracts S. 544, 608, 710) beschrieben, wobei die erzielten Effekte auch die Therapie von schweren Hyperlipidämien ermöglichen. Deshalb erscheint es als bedeutungsvoll, bei dem gegebenen Wirkprinzip geeignete Stoffe ohne die Nachteile der gegenwärtig verwendeten Präparate zu finden. Folgende Merkmale der genannten Präparate und insbesondere von Colestipol sind als verbesserungswürdig anzusehen:
1. Die hohen Tagesdosen, die notwendig sind, weil nur eine relativ geringe Bindungsrate bei neutralem pH in isotonem Medium besteht, sowie die (teilweise) Wiederfreisetzung der adsorbierten Gallensäuren.
2. Die qualitative Verschiebung der Gallensäurezusammensetzung der Galle mit abnehmender Tendenz für Chenodesoxycholsäure und die damit verbundene zunehmende Gefahr für Cholelithiasis.
3. Das Fehlen einer dämpfenden Wirkung auf den Cholesterinstoffwechsel der Darmbakterien.
4. Die zu hohe Bindungsrate von Vitaminen und Pharmaka macht einen Substitutionsbedarf an diesen Stoffen und Blutspiegelkontrollen eventuell notwendig.
5. Die Darreichungsform ist bislang als unzureichend anzusehen.

Aufgabe der Erfindung war es, eine Möglichkeit zu schaffen, Gallensäuren in konzentrationsabhängiger Weise zu binden, wobei die Gallensäuren bindenden Verbindungen selbst nicht mit resorbiert werden und damit nicht in den enterohepatischen Kreislauf gelangen. Ebenso sollen die Verbindungen eine hohe Bindungsrate für Gallensäuren bei neutralem pH aufweisen und gleichzeitig gewährleisten, daß diese unter physiologischen Bedingungen nicht wieder freigesetzt werden und somit nicht resorbiert werden können.

EP-A-389079, EP-A-27412 und EP-A-439846 offenbaren ähnliche Verbindungen, die aber nicht die oben angegebenen Eigenschaften der erfindungsgemäßen Verbindungen aufweisen.

Darüberhinaus sollen diese Verbindungen, die bestehenden Nachteile der bekannten "Resins" nicht bzw. nicht mehr im bekannten Ausmaß, aufweisen.

Die Lösung der Aufgabe besteht in der Bereitstellung von α- oder β-verknüpften Polyaspartamidderivaten der allgemeinen Formel I, 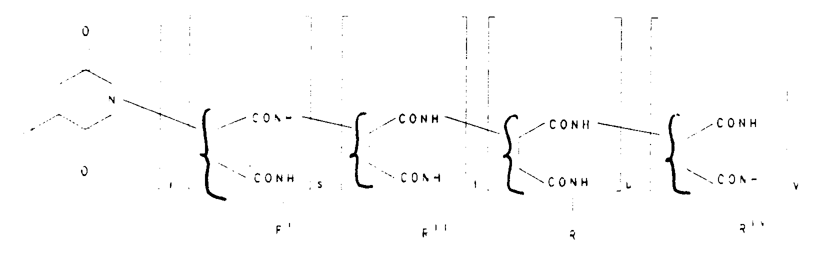 in der
- R^{I}, R^{II}, R^{III}, R^{IV}: gleich oder verschieden sind und H, ein Rest der allgemeinen Formel II

- A - N (R₁)(R₂), II

worin
A ein Alkylen-, Alkenylen- oder Alkinylenrest mit 2 bis 15, vorzugsweise 2 bis 6 C-Atomen, der geradkettig oder verzweigt ist, vorzugsweise geradkettig ist,
- R₁ und R₂: unabhängig voneinander Wasserstoff oder (C₁-C₁₈)-Alkyl, bevorzugt (C₁-C₃)-Alkyl,
ein Rest der allgemeinen Formel III

- A - OCOR₃, III

oder ein Rest der allgemeinen Formel IV

-A - OH, IV

worin
A die oben angegebene Bedeutung hat und
R₃ der Rest, der eine über ihre Carboxygruppe gebundene natürliche oder synthetische Fettsäure ist oder eine Dicarbonsäure bzw. eine Dicarbonsäureester oder -amid mit 2 - 8 C-Atomen, sowie
- r: 0 bis 0,5,
- s: 0,1 bis 0,9,
- t: 0,9 bis 0,1,
- u: 0 bis 0,5 und
- v: 0 bis 0,5 bedeutet,

Besonders bevorzugt ist, wenn
r oder u = Null ist oder die Summe aus r und u gleich Null ist.

Vorzugsweise ist R^{I} ein Rest der allgemeinen Formel II und R^{II} und R^{III} ein Rest der allgemeinen Formel III, sowie ggf. R^{IV} ein Rest der allgemeinen Formel IV.

Besonders bevorzugt sind erfindungsgemäße Verbindungen, in denen R^{II} und R^{III} verschiedene Reste der Formel III tragen.

Zu den Dicarbonsäurederivaten zählen Succinoyloxybutyl- und -hexylreste.

Auch sind verträgliche aromatische Carbonsäurereste, wie z. B. Zimtsäure oder Di-Hydrozimtsäure einsetzbar.

Von den Alkoholen, die die Esterkomponente in den Dicarbonsäureestern darstellen werden folgende eingesetzt: gesättigte und ungesättigte, lineare oder verzweigte Alkohole wie Hexanol, Octanol, Decanol, Dodecanol, Tetradecanol, Hexadecanol, Octadecanol, u. a. Ebenso sind auch solche darunter zu verstehen, die weitere OH-Gruppen tragen können, wie etwa 1,2-Hexandiol, 1,2-Dodecandiol oder 1,2-Hexadecandiol.

Die erfindungsgemäßen Verbindungen weisen im allgemeinen ein Molekulargewicht von 10³ bis 10⁶, vorzugsweise von 10⁴ bis 10⁵ auf.

Der zu erzielende Substitutionsgrad der Reste R^{I}, R^{II}, R^{III}, R^{IV}, bezogen auf eine Aspartamideinheit, ist abhängig vom eingesetzten Rest und von den gewählten Reaktionsbedingungen. Schließlich wird er in der Weise eingestellt, daß die zu erzielende pharmakologische Wirkung der erfindungsgemäßen Verbindungen optimal erreicht wird.

Im allgemeinen liegt der Substitutionsgrad DS zwischen 0,1 und 1,0, vorzugsweise zwischen 0,2 und 0,8 für R^{I} und R^{II}. Für R^{III} werden bevorzugt Werte zwischen 0,1 und 0,5 bestimmt.

Die Derivatisierung des Polyaspartamides mit den Verbindungen der Formeln II und III führt zu einer Lipophilisierung des Polymeren. Diese Lipophilisierung führt dazu, daß Gallensäuren in erhöhtem Maße und insbesondere bei einem physiologischen pH-Wert fest an das Polymere adsorptiv gebunden werden. Hierbei handelt es sich um eine Bindung, die aus einer ionischen und einer strukturellen Wechselwirkung zwischen den Gallensäuremolekül und dem Polyaspartamidmolekül besteht.

Gallensäuren liegen bei einem physiologischen pH-Wert ionisiert oder protoniert vor. Insofern ist eine ionische Wechselwirkung gegeben. Die strukturelle Wechselwirkung beruht darauf, daß Gallensäuren vom lipophilierten Polyaspartamidmolekül räumlich umgeben werden. Es handelt sich daher um Einschlußverbindungen.

Grundsätzlich ist es daher möglich, die Polyaspartamidderivate sowohl zu ionisieren (Aminosäure, Aminoalkoxyreste) wie zu lipophilisieren (Fettsäuren, Alkohole), wobei sich die adsorptiven Wirkungen verstärken können. Ebenso ist es möglich, Gallensäuren als Derivatisierungsreagentien für Polyaspartamide einzusetzen, und die Derivate als Adsorber für Gallensäure zu verwenden.

In diesem Fall ist der Substitutionsgrad für die kovalent gebundenen Gallensäuren 0,01 bis 1, bevorzugt 0,3 bis 0,8.

Als natürliche Aminosäuren sind genannt:
Glycin, L-Alanin, L-Valin, L-Leucin, L-Serin, L-Threonin, L-Lysin, L-Arginin, L-Asparagin, L-Glutamin, L-Phenylalanin, L-Tyrosin, L-Prolin, L-Tryptophan.

Als synthetische Aminosäuren werden Enantiomerengemische der natürlichen Aminosäuren, Homoaminosäuren, Isoaminosäuren, γ-Aminobuttersäure, α-Aminobuttersäure etc. bevorzugt.

Als Fettsäuren sind geeignet gesättigte und ungesättigte, geradkettige oder verzweigte Carbonsäuren wie etwa Capron-, Heptan-, Octan-, Decan-, Dodecan-, Tetradecan-, Hexadecan-, Octadecansäure, Ölsäure, Linolsäure, Linolensäure, 2-Methylhexansäure, u. ä.

Folgende Alkohole werden z.B. zur Lipophilierung eingesetzt: gesättigte und ungesättigte, lineare oder verzweigte Alkohole wie Hexanol, Octanol, Decanol, Dodecanol, Tetradecanol, Hexadecanol, Octadecanol, u. a. Ebenso sind auch solche darunter zu verstehen, die weitere OH-Gruppen tragen können, wie etwa 1,2-Hexandiol, 1,2-Dodecandiol oder 1,2-Hexadecandiol.

Folgende Aminoalkylreste der Formel A - N (R₁)(R₂) werden verwendet:
2-Aminoethanol, 3-Aminopropanol, N,N-Dimethyl- oder -diethyl-2-aminoethanol, N,N-Dimethyl- oder -diethyl-3-aminopropanol, 4-Aminobutanol, 6-Aminohexanol, 4-Aminobuttersäure, 6-Aminocapronsäure, u. a.

Folgende Gallensäuren können an die erfindungsgemäßen Polyaspartamide gebunden werden: Cholsäure, Desoxycholsäure, Lithocholsäure, Ursodesoxycholsäure, Chenodesoxycholsäure, sowie deren entsprechenden Taurin und Glycinkonjugate.

Durch den Einsatz der erfindungsgemäßen Verbindungen können die beschriebenen Mängel der auf dem Markt befindlichen in den enterohepatischen Kreislauf eingreifenden "Resins" vollständig beseitigt werden. Durch Inhibition der Gallensäurerückresorption mit Hilfe der erfindungsgemäßen Verbindungen im Dünndarm wird auf wesentlich effektivere Weise die im enterohepatischen Kreislauf befindliche Gallensäurekonzentration vermindert, so daß eine Senkung des Cholesterinspiegels im Serum erfolgt. Avitaminosen sind bei Anwendung der erfindungsgemäßen Verbindungen danach ebensowenig vorhanden, wie die Beeinflussung der Resorption anderer Arzneimittel oder auch die negative Wirkung auf die Darmflora, da die Gallensäurebindung an die erfindungsgemäßen Verbindungen außerordentlich stabil ist.

Durch Einsatz der erfindungsgemäßen Verbindungen kann die sonst übliche Dosierung der Resins erheblich gesenkt werden, die empfohlene Dosis beträgt 0.5 - 10 g/kg/Tag. Die bekannten Nebenwirkungen (Obstipation, Steratorrhoe) sind deshalb nicht beobachtet worden, d. h. die Fettverdauung wird durch die naturnahe Struktur der Polyaspartamide einerseits und durch den bekanntermaßen positiven Einfluß von sogenannten Ballaststoffen auf die Verdauung andererseits werden nicht negativ beeinflußt.

Wegen der hohen Affinität der erfindungsgemäßen Verbindungen zu Gallensäuren stellt sich, verglichen mit der hohen Tagesdosis der "Resins", das Problem der Dosierung und dadurch auch das der Compliance nicht mehr. Daneben wird die Compliance dadurch verbessert, daß die erfindungsgemäßen Polyaspartamide wasserlöslich sind und somit für die Formulierung keine Zuschläge wie z. B. Geschmacksverbesserer, Emulgatoren, Süßstoffe etc. benötigt werden.

Die Herstellung von Polysuccinimid (= Polyanhydroasparaginsäure) ist aus EP-A-0 439 846 (entsprechend US-Patentanmeldung Nr. 651 295) bekannt.

Gegenstand der Erfindung ist weiterhin der Konjugat aus dem erfindungsgemäßen Polyaspartamidderivat und mindestens einer Gallensäure, die infolge einer strukturellen Wechselwirkung adsorptiv an das Polyaspartamidderivat gebunden ist.

Weiterhin betrifft die Erfindung die Verwendung der erfindungsgemäßen Komplexe zur Herstellung eines Arzneimittels. Die Verbindungen werden in pharmakologisch unbedenklichen organischen Lösungsmitteln, wie ein- oder mehrwertigen Alkoholen wie z. B. Ethanol oder Glycerin, in Triacetin, Ölen, wie z. B. Diethylenglykoldimethylether, oder auch Polyethern, wie z. B. Polyethylenglykol, oder auch in Gegenwart anderer pharmakologisch unbedenklicher Polymerträger, wie z. B. Polyvinylpyrrolidon, oder anderen pharmazeutisch annehmbaren Zusatzstoffen wie Stärke, Cyclodextrin oder Polysacchariden gelöst oder suspendiert oder gemischt. Ferner können die erfindungsgemäßen Verbindungen in Kombination mit anderen Arzneistoffen gegeben werden.

Die erfindungsgemäßen Konjugate werden in verschiedenen Dosierungsformen verabreicht, vorzugsweise oral in Form von Tabletten, Kapseln oder Flüssigkeiten, wie z. B. auch Lebensmitteln oder Fruchtsäften.

Die erfindungsgemäßen Polyaspartamide können zudem in analytischen Verfahren zur gruppenselektiven Anreicherung von Gallensäuren aus biologischen Flüssigkeiten wie z. B. Plasma, Serum, Urin, Galle etc. angewendet werden.

### Bestimmung der Adsorberkapazität

Zur In-vitro-Testung der Adsorberkapazität der erfindungsgemäßen Verbindungen werden die folgenden Gallensäuren: 15,1 mg Cholsäure, 13,8 mg Desoxycholsäure, 13,8 mg Chenodesoxycholsäure, 13,2 mg Lithocholsäure, 17,1 mg Glykocholsäure, 15,8 mg Glykodesoxycholsäure, 16,6 mg Glykochenodesoxycholsäure und 16,0 mg Glykolithocholsäure in 700 µl Methanol gelöst, mit 0,92 ml Phosphat-gepufferter Kochsalzlösung (pH 7.2) versetzt und zusammen mit 5 mg der erfindungsgemäßen Verbindungen 24 h bei 37 °C im Schüttelwasserbad inkubiert. Danach wird die Mischung in einem Dialyseschlauch vom Typ Visking gefüllt und 72 h bei Raumtemperatur gegen Phosphat-gepufferte Kochsalzlösung (pH 7.2) dialysiert. Die Bestimmung der Gallensäurebindung erfolgt durch Analytik des Außenmediums, z. B. durch die im folgenden beschriebenen Methoden.

### 1) HPLC mit Fluoreszenzdetektion

- Geräte:: HPLC-Anlage der Fa. Kontron, bestehend aus drei Pumpen und Mischkammer, Autosampler, UV-Detektor und Auswerteeinheit mit Software MT2. Fluoreszenzdetektor der Firmen Merck und Hitachi. Da die Proben licht- und temperaturempfindlich sind, wird der Autosampler auf ca. 5 °C gekühlt.
- Mobile Phase:: Laufmittel A: ®Millipore-Wasser (eigene Anlage)
Laufmittel B: Acetonitril/Methanol 60 : 30
- Säule:: ®LiChrospher 100 RP-18, 25 mm, 5 µm, Fa. Merck
- Vorsäule:: LiChrospher 60 RP-select B, 4 mm, 5 µm, Fa. Merck
- Flußrate:: 1,3 ml/min
- Detektion:: Excitation: 340 nm
Emission: 410 nm
- Gradient:: 0.00 min 66 % B
7.50 min 66 % B
8.00 min 76 % B
12.50 min 76 % B
13.00 min 83 % B
25.00 min 83 % B
25.50 min 91 % B
40.00 min 91 % B

### 2) Enzymatische Bestimmung der Gesamtgallensäure

- In Eppendorfgefäße werden je 900 µl des folgenden Gemisches gegeben:
   6 ml Tetra-Natrium-Diphosphat-Puffer 0.1 M, pH 8.9
   2 ml NAD-Lösung (4 mg/ml Wasser)
   20 ml Millipore-Wasser
- Dazu werden 30 µl der Probe und 30 µl Enzymlösung pipettiert.
- Enzymlösung: 3-alpha-Hydroxysteroiddehydrogenase 0.5 units/ml
- Die Ansätze werden gemischt und 2 h bei Raumtemperatur inkubiert.
- Anschließend Umfüllen in 1 ml-Einmalküvetten und Messung im Photometer bei 340 nm.
- Für Gallensäureproben nur bedingt geeignet, da die grüne Farbe stört.

### 3) HPLC mit UV-Detektion

- Geräte:: HPLC-Anlage der Fa. Kontron, bestehend aus drei Pumpen und Mischkammer, Autosampler, UV-Detektor und Auswerteeinheit mit Software MT2.
- Mobile Phase:: Laufmittel A: Ammoniumcarbamatpuffer 0.019 M, mit Phosphorsäure auf pH 4,0 eingestellt.
Laufmittel B: Acetonitril
- Säule:: LiChrospher 100 RP-8, 25 mm, 5 µm, Fa. Merck
- Vorsäule:: LiChrospher 60 RP-select B, 4 mm, 5 µm, Fa. Merck
- Flußrate:: Gradient: 0.00 min 0.8 ml/min
20.00 min 0.8 ml/min
23.00 min 1.3 ml/min
51.00 min 1.3 ml/min
- Detektion:: 200 nm (für Präparate zusätzlich bei 254 nm)
- Gradient:: 0.00 min 32 % B
8.00 min 35 % B
17.00 min 38 % B
20.00 min 40 % B
24.00 min 40 % B
30.00 min 50 % B
45.00 min 60 % B

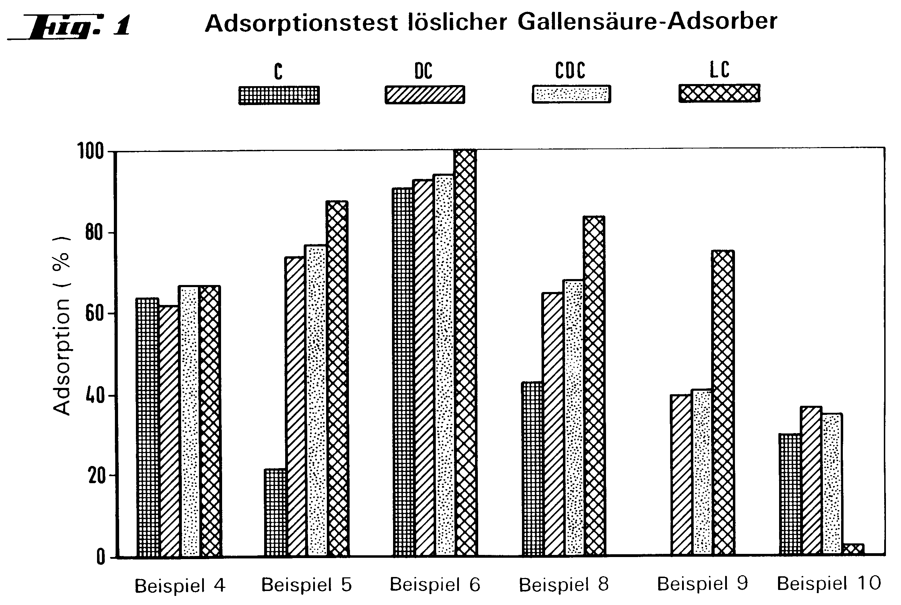

Die folgenden Ergebnisse konnten erhalten werden. Sie sind in Figur 1 zusammengefaßt. Figur 1 zeigt die Adsorptionseigenschaften der erfindungsgemäßen Polyaspartamidderivate bezüglich:
- C: Cholsäure
- CDC: Chenodesoxycholsäure
- DC: Desoxycholsäure
- LC: Lithocholsäure

Die Verbindungen wurden wie folgt hergestellt:

### Beispiel 1: Herstellung von Poly-α,β-(hydroxyethyl)-D,L-aspartamid-co-α,β-(dimethylaminoethyl)-D,L-aspartamid (40 : 60)

10 g (103 mmol) Polyanhydroasparaginsäure (M_{visc.} = 25000) werden in 80 ml DMF (wasserfrei) gegebenenfalls unter gelindem Erwärmen gelöst. Zu dieser Lösung werden zunächst 2,5 g (41 mmol) frisch destilliertes Dimethylaminoethylamin in 20 ml DMF verdünnt zugetropft sowie 1 g wasserfreies 2-Hydroxypyridin gegeben und einen Tag bei Raumtemperatur gerührt. 8,8 g (100 mmol) Aminoethanol werden anschließend zugefügt. Nach einem weiteren Tag Rühren wird für 2 h auf 50 °C erwärmt und das DMF bei 40 °C einrotiert. Der Rückstand wird in Wasser gelöst und über eine 10000 Membran ultrafiltriert. Das Retentat wird gefriergetrocknet und die Zusammensetzung des erhaltenen Copolymeren NMR-spektroskopisch überprüft.
Ausbeute: 9,5 g.
¹H-NMR (300 MHz) in D₂O/CF₃COOD:
   a) Singulett bei 2,79 ppm, -N(CH₃)₂
   b) breites Signal bei 2,66 ppm, CH₂ (Hauptkette)
   c) breite Signale bei 3,14 ppm und 3,46 ppm, 4H, CH₂ (Seitenketten)
   d) breite Signale um 4,2 ppm und 4,55 ppm, 1H, CH (Hauptkette).

### Beispiel 2: Herstellung von Poly-α,β-(hydroxyethyl)-D,L-aspartamid-co-α,β-(dimethylaminoethyl)-D,L-aspartamid (75 : 25)

15 g (155 mmol) Polyanhydroasparaginsäure (M_{visc.} = 19000) werden in 80 ml DMF (wasserfrei) gegebenenfalls unter gelindem Erwärmen gelöst. Zu dieser Lösung werden zunächst 3,40 g (39 mmol) frisch destilliertes Dimethylaminoethylamin in 20 ml DMF verdünnt zugetropft sowie 1 g wasserfreies 2-Hydroxypyridin gegeben und einen Tag bei Raumtemperatur gerührt. 10 g (164 mmol) Aminoethanol werden anschließend zugefügt. Nach einem weiteren Tag Rühren wird für 2 h auf 50 °C erwärmt und in wasserfreiem Aceton wiederholt ausgefällt.
Ausbeute: 24 g.

### Beispiel 3: Herstellung von Polyanhydroasparaginsäure-co-α,β-(hydroxyethyl)-D,L-aspartamid-co-α,β-(dimethylaminoethyl)-D,L-aspartamid (15 : 20 : 65)

10 g (103 mmol) Polyanhydroasparaginsäure werden in 80 ml DMF (wasserfrei) gegebenenfalls unter gelindem Erwärmen gelöst und mit 1 g Hydroxypyridin versetzt. Anschließend werden 5,9 g (67 mmol) Dimethylaminoethylamin, in 20 ml DMF gelöst, zugetropft und einen Tag bei Raumtemperatur gerührt. Danach werden 1,25 g (20,6 mmol) Aminoethanol, in 10 ml DMF verdünnt, zugegeben. Nach ebenfalls einem Tag bei Raumtemperatur wird zur Reaktionsvervollständigung 3 Stunden auf 60 °C erwärmt und in wasserfreiem Aceton wiederholt ausgefällt.

### Beispiel 4: Herstellung von Polyanhydroasparaginsäure-co-α,β-(palmitoyloxyethyl)-D,L-aspartamid-co-α,β-(dimethylaminoethyl)-D,L-aspartamid (15 : 20 : 65)

10 g Polyanhydroasparaginsäure-co-α,β-(hydroxyethyl)-D,L-aspartamid-co-α,β-(dimethylaminoethyl)-D,L-aspartamid (15 : 20 : 65) aus Beispiel 3 werden in 100 ml wasser- und aminfreiem DMF gelöst und mit 10 ml Palmitinsäurechlorid langsam versetzt. Anschließend werden 10 ml Pyridin, das eine Spatelspitze DMAP enthält, zugetropft und über Nacht gerührt. Zur Reaktionsvervollständigung wird anschließend für 2 Stunden auf 60 °C erwärmt. Der Ansatz wird mehrfach in wasserfreiem Aceton gefällt und im Vakuum getrocknet.

### Beispiel 5: Herstellung von Poly-α,β-(palmitoyloxyethyl)-D,L-aspartamid-co-α,β-(dimethylaminoethyl)-D,L-aspartamid (40 : 60)

10 g Poly-α,β-(palmitoyloxyethyl)-D,L-aspartamid-co-α,β-(dimethylaminoethyl)-D,L-aspartamid (40 : 60) aus Beispiel 1 werden in 100 ml wasser- und aminfreiem DMF gelöst und mit 15 ml Palmitinsäurechlorid versetzt. 15 ml Pyridin werden zugetropft sowie eine Spatelspitze DMAP zugegeben. Über Nacht wird gerührt und 2 Stunden auf 60 °C zur Reaktionsvervollständigung erwärmt. Die zunächst heterogene Reaktionsmischung wird dabei klar. Anschließend wird in Aceton gefällt, der Niederschlag getrocknet, in Wasser unter Erwärmung gelöst (trübe) und nach Filtrieren über eine 10000er Membran ultrafiltriert sowie anschließend das Retentat gefriergetrocknet.
Ausbeute: 10,7 g.

### Beispiel 6: Herstellung von Poly-α,β-(palmitoyloxyethyl)-D,L-aspartamid-co-α,β- (3-butyloxycarbonyl)propionyloxyethyl)-D,L-aspartamid-co-α,β-(dimethylaminoethyl)-D,L-aspartamid (15 : 60 : 25)

5 g Poly-α,β-(hydroxyethyl)-D,L-aspartamid-co-α,β-(dethylaminoethyl)-D,L-aspartamid (75 : 25) werden in 60 ml getrocknetem DMF gelöst. In einen Tropftrichter werden 1,25 g (1,4 ml) Palmitinsäurechlorid und 3,5 g 3-Butyloxycarbonyl-propansäurechlorid eingewogen und auf 10 ml mit trockenem DMF aufgefüllt. Die Lösung wird langsam zugetropft. Anschließend werden 2 ml Pyridin in 3 ml DMF gelöst und ebenfalls langsam zur Reaktionsmischung zugetropft. Nach 3 Stunden wird die Prozedur der Säurechlorid- und Pyridinzugabe wiederholt und der Ansatz über weitere 5 Tage gerührt. Der Ansatz wird dann in Diisopropylether gefällt, der Rückstand getrocknet und anschließend in Wasser gelöst. Über eine 10000er Membran wird ultrafiltriert sowie das Retentat gefriergetrocknet. Die Zusammensetzung muß NMR-spektroskopisch überprüft werden. Dazu werden folgende Signale herangezogen:
- 'H-NMR in D₂O:: breites Triplett um 0,85 ppm, CH₃ der Palmitoyl- und Butylgruppen,
breites Signal zwischen 1,05 und 2 ppm, eine CH₂-Gruppe aus dem Butoxyflügel und 13 CH₂-Gruppen aus dem Palmitoylrest, daraus läßt sich das Verhältnis von Butyloxycarbonyl-propionyloxyethylgruppen zu Palmitoylgruppen berechnen Singulett bei 3 ppm, (CH₃)N-,
daraus ergibt sich das Verhältnis von Ester- zu Aminogruppen

### Beispiel 7: Herstellung von Poly-α,β-(palmitoyloxyethyl)-D,L-aspartamid-co-α,β-(3-butyloxycarbonyl)propionyloxyethyl)-D,L-aspartamid-co-α,β-(dimethylaminoethyl)-D,L-aspartamid-co-α,β-(hydroxyethyl)-D,L-aspartamid (15 : 50 : 25 : 10)

5 g Poly-α,β-(hydroxyethyl)-D,L-aspartamid-co-α,β-(dimethylaminoethyl)-D,L-aspartamid (75 : 25) werden in 60 ml getrocknetem DMF gelöst. In einen Tropftrichter werden 1,25 g (1,4 ml) Palmitinsäurechlorid und 3,5 g 3-Butyloxycarbonyl-propansäurechlorid eingewogen und auf 10 ml mit trockenem DMF aufgefüllt. Die Lösung wird langsam zugetropft. Anschließend werden 2 ml Pyridin mit 3 ml DMF gemischt und ebenfalls langsam zur Reaktionsmischung zugetropft. Nach Rühren über Nacht wird der Ansatz dann in Diisopropylether gefällt, der Rückstand getrocknet und anschließend in Wasser gelöst. Über eine 10000er Membran wird ultrafiltriert sowie das Retentat gefriergetrocknet. Analytik analog Beispiel 6.

Im Gegensatz zu Beispiel 6 verläuft die Reaktion wesentlich unvollständiger und führt zu einem Produkt mit etwa 10 % unveresterten Hydroxylgruppen.

### Beispiel 8: Herstellung von α,β-Poly(2-dimethylaminoethyl)-co-(palmitoyloxyethyl)-D,L-aspartamid (80 : 20)

Die Herstellung erfolgt analog zu Beispiel 5.
Ausbeute: 10,2 g.

### Beispiel 9: Herstellung von α,β-Poly(2-dimethylaminoethyl)-co-(palmitoyloxyethyl)-D,L-aspartamid (40 : 60)

Die Herstellung erfolgt analog zu Beispiel 5.
Ausbeute: 10,6 g.

### Beispiel 10: Herstellung von α,β-Poly(2-dimethylaminoethyl)-co-(palmitoyloxyethyl)-D,L-aspartamid (20 : 80)

Die Herstellung erfolgt analog zu Beispiel 5.
Ausbeute: 10,5 g.

## Patentansprüche

1. α- oder β-verknüpfte Polyaspartamidderivate der allgemeinen Formel I 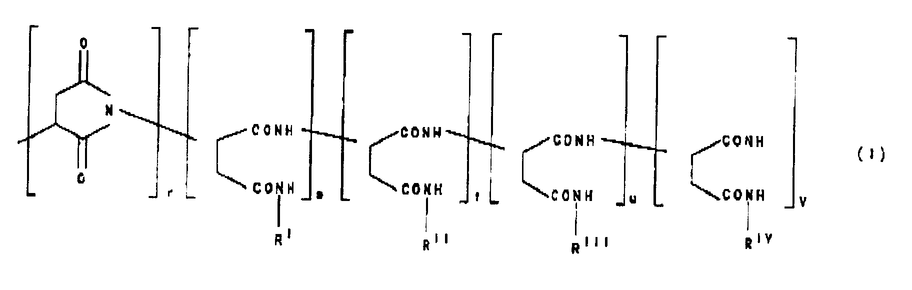 in der
R^{I}: H oder ein Rest der allgemeinen Formel II
-A-N(R₁)(R₂), II
worin
A ein Alkylen-, Alkenylen- oder Alklnylenrest mit 2 bis 15, vorzugsweise 2 bis 6 C-Atomen, der geradkettig oder verzweigt, vorzugsweise geradkettig ist,
R₁ und R₂ unabhängig voneinander Wasserstoff oder (C₁-C₁₈)-Alkyl, bevorzugt (C₁-C₃)-Alkyl,
und
R^{II}, R^{III}, R^{IV} : gleich oder verschieden sind und zwar H oder ein Rest der allgemeinen Formel III
-A-OCOR₃, III
worin
A die oben angegebene Bedeutung hat und
R₃ eine über ihre Carboxygruppe gebundene natürliche oder synthetische Fettsäure oder eine Dicarbonsäure bzw. eines Dicarbonsäureesters oder -amides mit 2 - 8 C-Atomen,
oder ein Rest der allgemeinen Formel IV
-A-OH, IV
worin
A die oben angegebene Bedeutung hat,
r 0 bis 0,5,
s 0,1 bis 0,9,
t 0,9 bis 0,1,
u 0 bis 0,5 und
v 0 bis 0,5.
bedeutet.

2. Verbindungen nach Anspruch 1, in denen die Summe aus r und u oder r oder u Null ist.

3. Verbindungen nach den Ansprüchen 1 bis 2, dadurch gekennzeichnet, daß R^{II} und R^{III} verschiedene Reste der Formel III tragen,

4. Verbindungen nach nach Ansprüche 1 bis 3, dadurch gekennzeichnet, daß sie ein Molekulargewicht von 10³ bis 10⁶, vorzugsweise von 10⁴ bis 10⁵ aufweisen.

5. Verbindungen nach den Ansprüchen 1 bis 4, dadurch gekennzeichnet, daß sie einen Substitutionsgrad DS zwischen 0,1 und 1,0 aufweisen

6. Konjugate bestehend aus einer Verbindung gemäß Ansprüchen 1 bis 5 und mindestens einer Gallensäure.

7. Arzneimittel, enthaltend ein Konjugat gemäß Anspruch 6 und einen Träger.

8. Verfahren zur Herstellung eines Arzneimittels, dadurch gekennzeichnet, daß man ein Arzneimittel nach Anspruch 7 in eine geeignete Dosierungsform überführt.

9. Verwendung der Konjugate gemäß Anspruch 6 zur Herstellung eines Arzneimittels zur Behandlung von Hypolipidämie.

10. Verwendung der Konjugate gemäß Anspruch 6 in Lebensmitteln und Fruchtsäften.

11. Verwendung der Verbindungen gemäß Ansprüchen 1 bis 5 zur nichttherapeutischen gruppenselektiven Anreicherung von Gallensäuren aus biologischen Flüssigkeiten.

## Claims

1. An α- or β-linked polyaspartamide derivative of the formula I 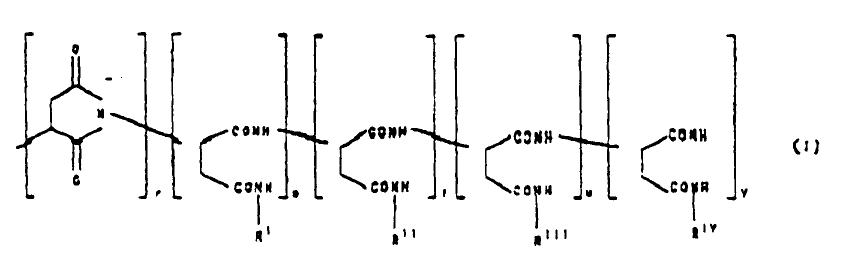 in which
R^{I} is H or a radical of the formula II
-A-N(R₁)(R₂) II
in which
A is an alkylene, alkenylene or alkynylene radical having 2 to 15, preferably 2 to 6, carbon atoms, which is straight-chain or branched, preferably straight-chain,
R₁ and R₂ independently of one another are hydrogen or (C₁-C₁₈)-alkyl, preferably (C₁-C₃)-alkyl,
and
R^{II}, R^{III} and R^{IV} are identical or different and are actually H or a radical of the formula III
-A-OCOR₃ III
in which
A has the abovementioned meaning and
R₃ is a natural or synthetic fatty acid bonded via its carboxyl group or a dicarboxylic acid or a dicarboxylic acid ester or amide having 2-8 carbon atoms,
or a radical of the formula IV
-A-OH IV
in which
A has the abovementioned meaning,
r is 0 to 0.5,
s is 0.1 to 0.9,
t is 0.9 to 0.1,
u is 0 to 0.5 and
v is 0 to 0.5.

2. A compound as claimed in claim 1, in which the sum of r and u or r or u is zero.

3. A compound as claimed in claim 1 or 2, wherein R^{II} and R^{III} carry various radicals of the formula III.

4. A compound as claimed in any of claims 1 to 3, which has a molecular weight of 10³ to 10⁶, preferably of 10⁴ to 10⁵.

5. A compound as claimed in any of claims 1 to 4, which has a degree of substitution DS between 0.1 and 1.0.

6. A conjugate comprising a compound as claimed in any of claims 1 to 5 and at least one bile acid.

7. A pharmaceutical comprising a conjugate as claimed in claim 6 and an excipient.

8. A process for the production of a pharmaceutical, which comprises converting a pharmaceutical as claimed in claim 7 into a suitable dosage form.

9. The use of the conjugate as claimed in claim 6 for the production of a pharmaceutical for the treatment of hypolipidemia.

10. The use of the conjugate as claimed in claim 6 in foodstuffs and fruit juices.

11. The use of the compounds as claimed in any of claims 1 to 5 for the non-therapeutic group-selective enrichment of bile acids from biological fluids.

## Revendications

1. Dérivés de polyaspartamide couplés en α ou β de formule générale I 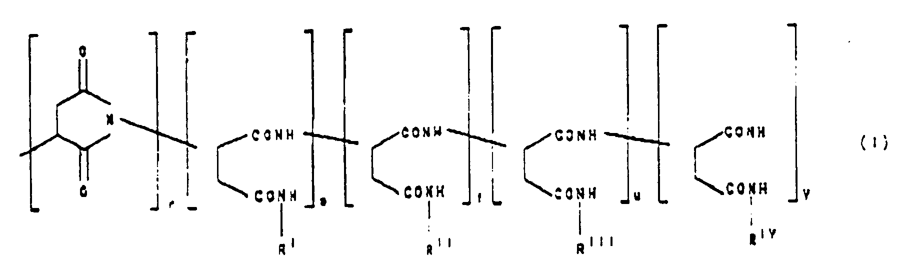 dans laquelle
R^{I} représente un atome d'hydrogène ou un groupe de formule générale II
-A-N(R₁)(R₂) II
dans laquelle
A est un groupe alkylène, alcénylène ou alcynylène avec 2 à 15, de préférence 2 à 6 atomes de carbone, qui est à chaîne linéaire ou ramifiée, de préférence linéaire,
R₁ et R₂ représentent indépendamment l'un de l'autre un atome d'hydrogène ou un groupe alkyle en C₁-c₁₈, de préférence un groupe alkyle en C₁-C₃,
et
R^{II}, R^{III}, R^{IV} sont identiques ou différents et représentent un atome d'hydrogène ou un groupe de formule générale III
-A-OCOR₃ III
dans laquelle
A a la signification indiquée ci-dessus et
R₃ représente un acide gras naturel ou synthétique lié par son groupe carboxy ou un acide dicarboxylique ou un ester ou un amide d'acide dicarboxylique avec 2 - 8 atomes de carbone,
ou un groupe de formule générale IV
-A-OH IV
dans laquelle
A a la signification indiquée ci-dessus,
r représente 0 à 0,5,
s 0,1 à 0,9,
t 0,9 à 0,1
u 0 à 0,5 et
v 0 à 0,5.

2. Composés selon la revendication 1, dans lesquels la somme de r et u ou r ou u est nul.

3. Composés selon les revendications 1 à 2, caractérisés en ce que R^{I} et R^{II} portent différents groupes de formule III.

4. Composés selon les revendications 1 à 3, caractérisés en ce qu'ils présentent une masse moléculaire de 10³ à 10⁶, de préférence de 10⁴ à 10⁵.

5. Composés selon les revendications 1 à 4, caractérisés en ce qu'ils présentent un degré de substitution DS entre 0,1 et 1,0.

6. Composés conjugués constitués d'un composé selon les revendications 1 à 5 et d'au moins un acide biliaire.

7. Médicament, contenant un composé conjugué selon la revendication 6 et un véhicule.

8. Procédé de préparation d'un médicament, caractérisé en ce qu'on transforme un médicament selon la revendication 7 en une forme de dosage appropriée.

9. Utilisation des conjugués selon la revendication 6 pour le traitement de l'hypolipidémie.

10. Utilisation des conjugués selon la revendication 6 dans des aliments et des jus de fruits.

11. Utilisation des composés selon les revendications 1 à 5 pour l'enrichissement de groupe non thérapeutique d'acides biliaires provenant de fluides biologiques.
